Europäisches Patentamt

European Patent Office

Office européen des brevets

(11) Veröffentlichungsnummer: **0 208 102**
**A2**

# (12) EUROPÄISCHE PATENTANMELDUNG

(21) Anmeldenummer: 86107060.5

(22) Anmeldetag: 23.05.86

(51) Int. Cl.⁴: **C 07 C 29/15**
**C 10 L 1/02, B 01 J 23/58**

(30) Priorität: 08.07.85 DE 3524317

(43) Veröffentlichungstag der Anmeldung:
14.01.87 Patentblatt 87/3

(84) Benannte Vertragsstaaten:
AT BE CH DE FR GB IT LI LU NL SE

(71) Anmelder: Union Rheinische Braunkohlen Kraftstoff
Aktiengesellschaft
Ludwigshafener Strasse o. Nr. Postfach 8
D-5047 Wesseling(DE)

(72) Erfinder: Röper, Michael, Dr.
Albert-Schweizer-Strasse 10
D-6706 Wachenheim(DE)

(72) Erfinder: Keim, Wilhelm, Prof., Dr.
Brüsseler Ring 99
D-5100 Aachen(DE)

(72) Erfinder: Seibring, Joachim, Dr.
Wiesenweg 5
D-6714 Lambsheim(DE)

(72) Erfinder: Kolle-Görgen, Georg, Dr.
Grünstadter Strasse 6
D-6710 Frankenthal(DE)

(54) Verfahren zur katalytischen Herstellung eines Alkoholgemisches mit erhöhtem Isobutanolgehalt.

(57) Die Erfindung betrifft ein katalytisches Verfahren und einen Katalysator, der $ZrO_2$ und/oder Ceroxid, Pd und/oder seine Verbindungen und wenigstens ein Oxid und/oder Hydroxid der Alkali- und Erdalkalimetalle sowie ggf. wenigstens ein(e) weitere(s) Oxid und/oder Metall und/oder Verbindung enthält, zur Herstellung eines Alkoholgemisches mit erhöhtem Isobutanolgehalt aus Synthesegas.

EP 0 208 102 A2

Wesseling, den 28.06.1985
NL-Dr.Hö/Esch      - 2115 -

Unser Zeichen: - UK 362 -

-1 -

## Verfahren zur katalytischen Herstellung eines Alkoholgemisches mit erhöhtem Isobutanolgehalt

Die Erfindung betrifft ein katalytisches Verfahren und einen Katalysator, der $ZrO_2$ und/oder Ceroxid, Pd und/oder seine Verbindungen und wenigstens ein Oxid und/oder Hydroxid der Alkali- und Erdalkalimetalle sowie ggf. wenigstens ein(e) weitere(s) Oxid und/oder Metall und/oder Metallverbindung enthält, zur Herstellung eines Alkoholgemisches mit erhöhtem Isobutanolgehalt aus Synthesegas.

Die Hydrierung von Kohlenmonoxid an alkalimodifizierten Methanolkatalysatoren zur Herstellung höherer Alkohole, vor allem Isobutanol, neben Methanol ist seit langem bekannt.
Unter der Bezeichnung "Isobutylölsynthese" wurde dieses Verfahren 1935-52 von der BASF großtechnisch zur Gewinnung von Isobutanol durchgeführt.
Hierbei hat sich nur der mit 1 % KOH alkalisierte $ZnO/Cr_2O_3$-Katalysator bewährt. Durch andere Zusätze, z.B. Manganoxid, wurden teilweise sehr komplexe hochkondensierte Produkte erhalten. (s. z.B. A. Palm in Chem. Technologie III. Organische Technologie I, K. Winnacker, L. Küchler, Carl Hauser Verlag, München, 1971, S. 350 und dito 1981, S. 572)

Auch in den Offenlegungsschriften DE-OS 3 244 313, DE-OS 31 36 088 und DE-OS 31 19 290 der Anmelderin Snamprogetti S.p.A. wird ein nach einem speziellen Herstellungsverfahren gewonnener $ZnO/Cr_2O_3$-Katalysator offenbart.
In DE-OS 30 05 551 der Anmelderin Südchemie AG wird ein $Cu/ZnO/K_2O$ Katalysator beschrieben mit $Al_2O_3$ und $Cr_2O_3$ als Promotoren.

Der Gesamtanteil aller C$_4$-Alkohole beträgt bei diesem Verfahren 11,3 Gew.%.

In EP-A- 0 005 492 der Anmelderin Standard Oil Company wird ein Katalysator beschrieben, der neben ThO$_2$, Kupferoxid als Hauptbestandteil enthält. Als Promotoren werden Palladium und Natrium verwendet.

In den Offenlegungsschriften DE-OS 29 49 952 und DE-OS 33 10 540 der Anmelderin Institut Francais du Pétrole werden Kobalt/ Kupfer-Katalysatoren beschrieben.

Ein neues technisches Verfahren zur Herstellung eines Alkoholgemisches mit ca. 6-7 Gew.% Butanolen ist das Octamix-Verfahren der Fa. Lurgi. Als Katalysator wird ein modifizierter Niederdruckmethanol-Katalysator verwendet. (Erdöl und Kohle, -Erdgas- Petrochemie 38 1985, S. 19)

Da die Herstellung höherer Alkohole aus Synthesegas zunehmende Bedeutung gewinnt, insbesondere im Hinblick auf deren Einsatz als Kraftstoff bzw. Kraftstoffzusatz, wobei das Isobutanol aufgrund seiner Klopffestigkeit eine besonders interessante Komponente ist, lag der vorliegenden Erfindung die Aufgabe zugrunde, ein Verfahren und einen Katalysator zu entwickeln, die zu einer weiteren Erhöhung des Isobutanolanteils in einem aus Synthesegas erzeugten Alkoholgemisch führen.

Diese Aufgabe wurde erfindungsgemäß dadurch gelöst, daß die Herstellung des Alkoholgemisches in Gegenwart eines Katalysators erfolgt, der

a) Zirkonoxid und/oder Ceroxid in einer Menge von 25 - 99,998 Gew.%, bevorzugt von 50 - 99,9 Gew.%,

b) einen Anteil von metallischen Palladium und/oder Palladium in Form seiner Verbindungen von 0,001 - 5 Gew.%, bevorzugt von 0,005 - 3 Gew.% und

c) wenigstens ein Oxid und/oder Hydroxid aus der Gruppe der
   Alkali- und Erdalkalimetalle in einer Menge von 0,001 - 9
   Gew.%, vorzugsweise von 0,1 - 5 Gew.%
enthält.

Katalysator und Verfahren können noch weiter dadurch verbessert
werden, daß zusätzlich wenigstens ein(e) Oxid und/oder Metall
und/oder Verbindung aus der Gruppe
B, Al, Ga, In, Tl, Si, Ge, Sn, Pb, Cu, Zn, Se, Y, La, Ti, Hf,
Th, V, Nb, Ta, Cr, Mo, W, Mn, Fe, Co, Ni, Ru, Rh, Os, Ir, Pt,
seltene Erden mit Ausnahme von Cer,
im Katalysator enthalten ist.

Als besonders geeignet hat sich als Zusatz Manganoxid erwiesen.
In den erfindungsgemäßen Katalysatoren liegt das Zirkonoxid
in einer Menge von 25 - 99,998, bevorzugt mit 50 - 99,9 Gew.%
vor, Palladium in einer Menge von 0,001 - 5 Gew.%, bevorzugt
0,005 - 3 Gew.% und Alkali- und/oder Erdalkalidioxid und/oder Hydroxid in einer Menge von 0,001 - 9, vorzugsweise 0,1 - 5 Gew.% vor.
Zusätzliche oxidische oder metallische Verbindungen können in
Mengen von 0,001 bis 74,9 Gew.% enthalten sein, wobei im Falle
oxidischer Zusätze bevorzugt 1 - 40 Gew.% enthalten sind.
Diese können beispielsweise durch Fällen von wässrigen Metallsalzlösungen, z.B. Zirkonnitrat mit der gewünschten Basenlösung
erfolgen, wobei gleichzeitig andere gelöste Metallsalze ebenfalls der Fällung unterworfen werden können. Anschließend wird
das gefällte Material gewaschen, geformt und getrocknet und
schließlich zur Aktivierung reduzierend mit Wasserstoff behandelt und gegebenenfalls kalziniert.
Werden Träger verwendet, so kann nach Aufbringen der gewünschten Komponenten durch Tränken ebenfalls eine Aktivierung durch
Kalzinieren durchgeführt werden. Ferner kann dem Fällungsgemisch ein Bindemittel zugegeben werden, wobei durch gezielte
Zersetzung oder durch Abbrennen eine bestimmte Porengröße erzeugt werden kann.

Das Aufbringen des Palladiums erfolgt gewöhnlich durch Imprägnieren des Katalysators mit gelösten Palladiumsalzen, z.B. von Palladiumnitrat in Wasser, ebenso kann die Anreicherung des Alkali- bzw. Erdalkaligehalts durch Imprägnieren bzw. Tränken erfolgen sowie das Aufbringen zusätzlicher Metalle bzw. deren Verbindungen wie beispielsweise Ruthenium, Rhenium, Rhodium u.a. Auch durch Waschen des gefällten Materials mit den entsprechenden gelösten Basen kann eine solche Anreicherung erfolgen.
Das Aufbringen des Palladiums kann beispielsweise auch durch Lösen organischer Palladiumsalze, z.B. von Palladiumacetylacetonat in einem organischen Lösungsmittel oder einem Gemisch von Wasser mit einem polaren organischen Lösungsmittel, wie z.B. von Acetonitril erfolgen und Behandeln des Katalysators mit dieser Lösung, wobei das Salz nach dem Aufbringen thermisch zerstört werden kann.
Aufgebrachte Salze können auch beispielsweise durch Behandeln mit Formaldehyd, sowie hydrierend zerstört werden.
Die beschriebenen Herstellungsmethoden sind beispielhaft, jedoch nicht limitierend. Auch andere bekannte Herstellungsmethoden sind für die Herstellung der erfindungsgemäßen Katalysatoren geeignet.

Als Einsatzgas kann das übliche Synthesegas eingesetzt werden, das überwiegend $H_2$ und CO enthält, jedoch auch $CO_2$ und andere Gase enthalten kann. Bekanntlich erhöht sich aus stöchiometrischen Gründen bei relativ hohem CO-Anteil der Anteil höherer Alkohole im Produkt. Ein geeignetes Gasgemisch enthält beispielsweise CO : $H_2$ im Verhältnis 1 : 1. Das Verhältnis kann jedoch in weiten Grenzen, nämlich 1 : 10 bis 10 : 1 variieren.
Die Reaktionstemperaturen können bei 200 - 500 °C liegen, bevorzugt jedoch bei 300 - 450 °C. Der Druck kann bei 10 - 600 bar, bevorzugt bei 20 - 400 bar liegen.
Die GHSV (Gas Hourly Space Velocity) kann 1000 - 100.000 $h^{-1}$, bevorzugt 5000 - 40.000 $h^{-1}$ betragen.

In Tabelle 1 sind Ergebnise dargestellt, die nach Verfahren des geschilderten Standes der Technik erhalten werden. Angegeben sind Druck, Temperatur, GHSV, die entstandenen Alkohole sowie Raum/Zeit-Ausbeute (RZA) in g pro Liter Katalysator und Stunde.

Der Isobutanolanteil liegt bei 11,2 - 18,1 Gew.%, die RZA bei 100 - 180.

Tab. 1

| | Snamprogetti $ZnO/Cr_2O_3/K_2O$ | Südchemie $Cu/ZnO/Al_2O_3/Cr_2O_3$ $K_2O$ | BASF $ZnO/Cr_2O_3$ $K_2O$ |
|---|---|---|---|
| Druck bar | 80 - 90 | 100 | 325 |
| Temperatur °C | 400 | 350 | 420 - 460 |
| GHSV $h^{-1}$ | 9.500 | 2.600 | 10.000-20.000 |
| Methanol Gew.% | 57,7 | 65,0 | 69,4 |
| Ethanol/ Propanol Gew.% | 3,4 | 14,8 | 4,2 |
| i-Butanol Gew.% | 18,1 | 11,2 | 15,4 - 19,4 |
| $C_5^+$-Alkohole | 20,8 | 9,0 | 11 - 7 |
| Raum/Zeit * Ausbeute g/l · h | 180 | 158 | |

\* Gesamtflüssigprodukt

In Tabelle 2 ist die Zusammensetzung einiger erfindungsgemäßer Katalysatoren angegeben.

Tab. 2

|       | $ZrO_2$ Gew.% | $K_2O$ Gew.% | MnO Gew.% | Pd Gew.% | $In_2O_3$ Gew.% |
|-------|---------------|--------------|-----------|----------|-----------------|
| Z 5   | 99,0          | 1,0          |           |          |                 |
| Z 9   | 99,0          | 0,9          |           | 0,1      |                 |
| ZJ 3  | 91,5          | 1            |           | 1,5      | 6               |
| ZMO   | 81,4          | 0,8          | 17,8      |          |                 |
| ZM 1  | 79,6          | 0,7          | 19,59     | 0,11     |                 |
| K 4   | 75,6          | 0,5          | 22,25     | 1,45     |                 |
| ZM 4  | 79,6          | 0,7          | 18,18     | 1,52     |                 |
| ZM 3  | 79,6          | 0,7          | 18,6      | 1,1      |                 |
| C 2   | 97,85 $Ce_2O_3$ | 0,7        |           | 1,45     |                 |
| CM 1  | 75,7 $Ce_2O_3$  | 0,7        | 22,15     | 1,45     |                 |

Die in Tabelle 3 zusammengefaßten Ergebnisse wurden in kontinuierlichen ca. 500 Stunden dauernden Versuchen ermittelt, wobei bei Beendigung der jeweiligen Versuche kein Nachlassen der Aktivität der Katalysatoren bezüglich Umsatz und Selektivität feststellbar war.

| Tabelle 3 | | | | | | | | | | | | | |
|---|---|---|---|---|---|---|---|---|---|---|---|---|---|
| | Z 5 | Z 9 | Z 9 | ZJ3 | ZMO | ZM1 | K4 | K4 | K4 | ZM4 | ZM3 | C2 | CM1 |
| Druck bar | 250 | 250 | 250 | 250 | 250 | 250 | 250 | 150 | 250 | 250 | 250 | 250 | 250 |
| Tempe- ratur °C | 420 | 420 | 420 | 420 | 420 | 420 | 420 | 420 | 420 | 420 | 420 | 420 | 420 |
| GHSV $h^{-1}$ | 11000 | 6700 | 12200 | 9750 | 18400 | 14600 | 13600 | 13900 | 21100 | 13000 | 11700 | 14200 | 14200 |
| Metha- nol Gew.% | 52,6 | 52,7 | 55,8 | 88,9 | 64,2 | 54,0 | 44,9 | 40,4 | 56,8 | 70,0 | 59,6 | 56,3 | 46,2 |
| Ethanol Propa- nol Gew.% | 2,4 | 1,4 | 1,1 | | 1,8 | 1,8 | | | | | 1,3 | | 1,1 |
| Isobu- tanol Gew.% | 27,7 | 29,1 | 26,4 | 11,1 | 23,5 | 27,9 | 39,7 | 43,6 | 30,2 | 23,8 | 27,8 | 28,7 | 37,0 |
| $C_5^+$-Alko- hole Gew.% | 17,3 | 16,8 | 16,7 | | 10,5 | 16,3 | 15,4 | 16,0 | 13,0 | 6,2 | 11,3 | 14,5 | 15,7 |
| Raum/Zeit Ausbeute (Alkohole) g/l . h | 132 | 220 | 296 | 103 | 680 | 648 | 706 | 392 | 1059 | 445 | 620 | 290 | 652 |

- 7 -

- UK 362 -

Aus Tabelle 3 werden die überlegenen Eigenschaften der erfindungsgemäßen Katalysatoren deutlich.

Katalysator Z 5, der nur aus $ZrO_2$ und $K_2O$ besteht, besitzt zwar eine erhebliche Selektivität bezüglich Isobutanol, jedoch wird eine nur geringe RZA erhalten.

Der Zusatz von nur 0,1 Gew.% Pd (Z 9) liefert eine Erhöhung der Isobutanolselektivität sowie eine 1,7-fache RZA bei einer GHSV von 6700 $h^{-1}$ und die 2,2-fache bei einer GHSV von 12.200 $h^{-1}$.

Der Katalysator ZJ3, der zusätzlich Indiumoxid enthält, liefert zwar eine relativ geringe RZA, jedoch eine überraschende Selektivität bezüglich Isobutanol. Wie die Tabelle zeigt, werden außer Isobutanol keine höheren Alkohole gebildet.

Der Katalysator ZMO enthält Manganoxid, jedoch kein Palladium, Auch dieser Katalysator besitzt eine höhere Selektivität als die des Standes der Technik und eine wesentlich höhere RZA. Durch Zugabe von nur 0,11 Gew.% Pd (ZM 1) steigt die Selektivität von 23,5 auf 27,9 Gew.% an.

Erhöht man die Pd-Menge auf über 1 Gew.% (K 4), so läßt sich die Selektivität auf 39,7 Gew.% steigern, begleitet von einer weiteren Erhöhung der RZA.

Durch Verringern des Druckes von 250 auf 150 bar geht zwar die RZA zurück, die Selektivität steigt jedoch auf 43,6 Gew.% Isobutanol an.

Erhöht man die GHSV auf 21000 $h^{-1}$, so erhöht sich die RZA auf 1059 g/l h bei einer noch immer hohen Selekvität von 30,2. Ähnliche Ergebnisse werden mit den Katalysatoren ZM4 und ZM3 erhalten, in denen die Pd-Menge variiert ist.

Die Ceroxid enthaltenden Katalysatoren C 2 und CM 1 liefern ebenfalls hervorragende Ergebnisse.

In Tabelle 4 sind Druck und Temperatur weiter variiert.

Tab. 4

ZM 3

| Druck<br>bar | 250 | 250 | 250 | 325 | 250 | 250 | 250 |
|---|---|---|---|---|---|---|---|
| Temperatur<br>°C | 420 | 440 | 400 | 420 | 420 | 440 | 400 |
| GHSV<br>$h^{-1}$ | 11.700 | 11.700 | 11.700 | 11.500 | 18.500 | 18.500 | 18.500 |
| Methanol<br>Gew.% | 59,6 | 33,2 | 57,4 | 57,9 | 63,9 | 41,1 | 67,8 |
| Ethanol/<br>Propanol<br>Gew.% | 1,3 | 2,3 | 4,3 | 2,0 | 1,0 | 14,2 | 0,91 |
| Isobuta-<br>nol<br>Gew.% | 27,8 | 38,3 | 22,3 | 27,1 | 24,4 | 29,1 | 21,9 |
| $C_5^+$-Alko-<br>hole<br>Gew.% | 11,3 | 26,2 | 16,0 | 13,0 | 10,7 | 15,6 | 9,4 |
| Raum/Zeit<br>Ausbeute<br>(Alkohole)<br>g/l· h | 620 | 474 | 543 | 722 | 990 | 684 | 751 |

Erwartungsgemäß erhält man bei Druckanstieg eine Erhöhung der
RZA. Die Versuche zeigen ferner, daß mit zunehmender Temperatur
die Selektivität zu Isobutanol ansteigt.

Auch bei weiterer Variation der Reaktionsbedingungen über die
Angaben in den Tabellen 3 und 4, die nicht als limitierend anzusehen sind, hinaus werden sehr gute Ergebnisse mit den erfindungsgemäßen Katalysatoren erhalten.

Obgleich die Versuche bei einem CO / $H_2$-Verhältnis von 1 : 1 durchgeführt wurden, ist dem Fachmann bekannt, daß man durch Variation dieses Verhältnisses den Anteil der höheren Alkohole beeinflussen kann. Dies gilt auch für die erfindungsgemäßen Katalysatoren. Es ist bekannt, daß durch Erhöhen dieses Verhältnisses der Anteil an höheren Alkoholen zunimmt, wobei der stöchiometrisch aus CO freiwerdende Sauerstoff überwiegend als $CO_2$ anfällt, anstelle von Wasser.

Dies kann bei Einsatz des alkoholischen Produkts dann von Interesse sein, wenn das Alkoholgemisch als sog. Fuel-Alkohol dem Benzin zugesetzt werden soll.

In Tabelle 5 sind weitere erfindungsgemäße Katalysatoren zusammengestellt, die neben $ZrO_2$ und $MnO_2$ zusätzliche Metalloxide sowie weitere Edelmetalle enthalten.

Tab. 5

| | $ZrO_2$ Gew.% | Alkali-/ Erdalka- lioxid Gew.% | $MnO_2$ Gew.% | Palla- dium Gew.% | zusätzliche Oxide Gew.% |
|---|---|---|---|---|---|
| K 41 | 75,8 | 0,5 $K_2O$ | 17,15 | 1,45 Pd | 5,1 $In_2O_3$ |
| K 411 | 75,8 | 0,5 $K_2O$ | 17,15 | 1,45 Pd | 5,1 $Fe_2O_3$ |
| K 412 | 75,8 | 0,5 $K_2O$ | 22,25 | 1,0 Pd 0,45 Pt | |
| K 413 | 75,8 | 0,5 $K_2O$ | 17,15 | 1,45 Pd | 5,1 CuO |
| K 414 | 75,8 | 0,5 $K_2O$ | 17,15 | 1,45 Pd | 3,5 $Cr_2O_3$ 1,6 $Fe_2O_3$ |
| K 42 | 75,8 | 0,5 $Na_2O$ | 17,15 | 1,45 Pd | 2,6 $Ce_2O_3$ 2,5 $Cr_2O_3$ |
| K 43 | 75,8 | 0,5 $K_2O$ | 22,75 | 1,2 Rh 0,25 Pd | |
| K 45 | 75,8 | 0,5 $Na_2O$ | 17,15 | 1,45 Pd | 51, $Cr_2O_3$ |
| K 46 | 75,8 | 0,5 $K_2O$ | 5,1 | 1,45 Pd | 17,15 $Cr_2O_3$ |
| K 47 | 75,8 | 0,5 CaO | 22,75 | 1,45 Pd | |
| K 48 | 75,8 | 0,7 $K_2O$ | 16,95 | 1,45 Pd | 3,5 $In_2O_3$ 1,6 $Al_2O_3$ |
| K 49 | 75,8 | 0,5 $K_2O$ | 17,15 | 1,45 Pd | 5,1 $La_2O_3$ |

Tab. 6

| | K41 | K411 | K412 | K413 | K414 | K42 | K43 | K44 | K45 | K46 | K47 | K48 | K49 | K4 |
|---|---|---|---|---|---|---|---|---|---|---|---|---|---|---|
| Druck bar | 250 | 250 | 250 | 60 | 250 | 250 | 250 | 250 | 250 | 250 | 250 | 250 | 250 | 250 |
| Temperatur °C | 420 | 420 | 420 | 280 | 420 | 420 | 420 | 420 | 420 | 420 | 420 | 420 | 420 | 420 |
| GHSV $h^{-1}$ | 13600 | 13600 | 13600 | 13600 | 13600 | 13600 | 13600 | 13600 | 13600 | 13600 | 13600 | 13600 | 13600 | 13600 |
| Methanol Gew.% | 45,9 | 49,1 | 43,9 | 44,9 | 48,2 | 45,1 | 44,5 | 49,3 | 52,7 | 59,4 | 45,3 | 46,3 | 48,7 | 44,7 |
| Ethanol Propanol Gew.% | | 2,1 | | | 0,5 | 0,2 | 0,3 | 0,2 | 0,7 | 1,7 | | | 3,1 | |
| Isobutanol Gew.% | 36,5 | 33,6 | 40,4 | 37,4 | 34,6 | 37,3 | 38,5 | 35,1 | 29,5 | 21,5 | 36,8 | 35,6 | 32,1 | 38,0 |
| $C_5^+$-Alkohole Gew.% | 17,6 | 15,2 | 15,7 | 17,7 | 16,7 | 17,4 | 16,7 | 15,4 | 17,1 | 17,4 | 17,9 | 18,1 | 16,1 | 17,3 |
| Raum/Zeit Ausbeute (Alkohole) g/l · h | 702 | 650 | 743 | 365 | 677 | 721 | 682 | 667 | 642 | 604 | 685 | 698 | 700 | 706 |

Die Versuchsergebnisse mit diesen Katalysatoren enthält Tabelle 6.

In Tabelle 7 sind einige Katalysatoren zusammengefaßt, die neben $ZrO_2$ zusätzliche Oxide ohne Manganoxid enthalten.

Tab. 7

| | $ZrO_2$ Gew.% | Alkali-/ Erdalkali- oxid Gew.% | Oxid I Gew.% | Palla- dium Gew.% | Oxid II[+] Gew.% |
|---|---|---|---|---|---|
| K 50 | 79,6 | 0,7 $K_2O$ | 18,25 $ThO_2$ | 1,45 | |
| K 51 | 79,6 | 0,7 $K_2O$ | 13,15 $ThO_2$ | 1,45 | 5,1 CuO |
| K 52 | 79,6 | 0,7 $K_2O$ | 13,15 $ThO_2$ | 1,45 | 3,5 CuO 1,6 ZnO |
| K 53 | 79,6 | 0,7 $K_2O$ | 18,25 CuO | 1,45 | |
| K 54 | 79,6 | 0,7 $K_2O$ | 13,15 ZnO | 1,45 | 5,1 $Cr_2O_3$ |
| K 55 | 79,6 | 0,7 $K_2O$ | 13,15 ZnO | 1,45 | 3,5 $CrO_3$ 1,6 CuO |
| K 56 | 79,6 $Ce_2O_3$ | 0,7 $Rb_2O$ | 18,25 ZnO | 1,45 | |
| K 57 | 79,6 | 0,7 $K_2O$ | 18,25 MgO | 1,45 | |

Tabelle 8 gibt die Versuchsergebnisse wieder. Es wird deutlich, daß die erfindungsgemäßen Katalysatoren auf Basis: Zirkonoxid, Alkali/Erdalkali und Pd auch in Abwesenheit von Manganoxid sehr gute Ergebnisse liefern.

Tab. 8

| | K 50 | K 51 | K 52 | K 53 | K 54 | K 55 | K 56 | K 57 |
|---|---|---|---|---|---|---|---|---|
| Druck bar | 250 | 60 | 60 | 60 | 250 | 80 | 250 | 250 |
| Tempe-ratur °C | 420 | 280 | 280 | 280 | 420 | 280 | 420 | 420 |
| GHSV h$^{-1}$ | 13.600 | 13.600 | 13.600 | 13.600 | 13.600 | 13.600 | 13.600 | 13.600 |
| Methanol Gew.% | 44,3 | 45,0 | 44,8 | 46,1 | 43,4 | 43,2 | 45,2 | 44,0 |
| Ethanol/ Propanol Gew.% | | 2,1 | 1,8 | 1,4 | 1,7 | 1,5 | 3,0 | 1,8 |
| Isobuta-nol Gew.% | 39,0 | 38,2 | 35,3 | 35,1 | 39,0 | 39,2 | 35,4 | 37,1 |
| $C_5^+$-Alko-hole Gew.% | 16,7 | 14,7 | 18,1 | 17,4 | 15,9 | 16,1 | 16,4 | 17,1 |
| Raum/Zeit Ausbeute (Alkohole) g/l · h | 702 | 373 | 320 | 585 | 690 | 605 | 693 | 679 |

Aus den Ergebnissen wird deutlich, daß der Zusatz weiterer Oxide, sowie die Verwendung zusätzlicher Edelmetalle ebenfalls zu hervorragenden Ergebnissen führt.

Insbesondere lassen sich in Gegenwart von Kupfer und seinen Verbindungen auch bei relativ milden Bedingungen sehr gute Ergebnisse erzielen.

Mit einem typischen erfindungsgemäßen Rohalkoholgemisch wurden Oktanzahlen ermittelt. Die erhaltenen Werte

$$ROZ = 108$$
$$MOZ = 91$$

liegen um nur zwei bzw. einen Punkt unter dem des reinen Isobutanols. Das erfindungsgemäße Reaktionsprodukt besitzt daher hervorragende Eigenschaften als "Fuel Methanol".

Verfahren zur katalytischen Herstellung
eines Alkoholgemisches mit erhöhtem
Isobutanolgehalt


PATENTANSPRÜCHE


1. Verfahren zur katalytischen Herstellung eines Alkoholgemisches mit erhöhtem Isobutanolgehalt aus CO und $H_2$ oder
   CO und $H_2$ enthaltenden Gasen, dadurch gekennzeichnet, daß
   die Herstellung des Alkoholgemisches in Gegenwart eines
   Katalysators erfolgt, der

   a) Zirkonoxid und/oder Ceroxid in einer Menge von 25 -
      99,998 Gew.%, bevorzugt von 50 - 99,9 Gew.%,

   b) einen Anteil von metallischen Palladium und/oder
      Palladium in Form seiner Verbindungen von 0,001 - 5
      Gew.%, bevorzugt von 0,005 - 3 Gew.% und

   c) wenigstens ein Oxid und/oder Hydroxid aus der Gruppe der
      Alkali- und Erdalkalimetalle in einer Menge von 0,001 -
      9 Gew.%, vorzugsweise von 0,1 - 5 Gew.% enthält.


2. Verfahren nach Anspruch 1, dadurch gekennzeichnet, daß die
   Herstellung des Alkoholgemisches in Gegenwart eines Katalysators durchgeführt wird, der zusätzlich wenigstens ein
   Oxid und/oder wenigstens ein Metall und/oder wenigstens
   eine Verbindung aus der Gruppe
   B, Al, Ga, In, Tl, Si, Ge, Sn, Pb, Cu, Zn, Sc, Y, La, Ti,
   Hf, Th, V, Nb, Ta, Cr, Mo, W, Mn, Fe, Co, Ni, Ru, Rh, Os,
   Ir, Pt, seltene Erden mit Ausnahme von Cer,
   in einer Menge von 0,001 - 74,9 Gew.% enthält.

3. Verfahren nach den Ansprüchen 1 und 2, dadurch gekennzeichnet, daß der Katalysator Zirkonoxid enthält.

4. Verfahren nach den Ansprüchen 1 bis 3, dadurch gekennzeichnet, daß der Katalysator Mangan und/oder Manganoxid und/oder andere Manganverbindungen enthält.

5. Verfahren nach den Ansprüchen 1 - 4, dadurch gekennzeichnet, daß der Katalysator Indium und/oder Indiumoxid und/oder andere Indiumverbindungen enthält.

6. Verfahren nach den Ansprüchen 1 - 5, dadurch gekennzeichnet, daß der Katalysator Kupfer und/oder Kupferoxid und/oder andere Kupferverbindungen enthält.

7. Verfahren zur katalytischen Herstellung eines Alkoholgemisches mit erhöhtem Isobutanolgehalt, aus CO und $H_2$ oder CO und $H_2$ enthaltenden Gasen, dadurch gekennzeichnet, daß die Herstellung des Alkoholgemisches in Gegenwart eines Katalysators erfolgt, der

a) Zirkonoxid und/oder Ceroxid in einer Menge von 25 - 99,998 Gew.%, bevorzugt von 50 - 99,9 Gew.%,

b) wenigstens ein Oxid und/oder Hydroxid aus der Gruppe der Alkali- und Erdalkalioxide in einer Menge von 0,001 - 9 Gew.%, bevorzugt von 0,1 - 5 Gew.% und

c) Mangan und/oder Manganoxid und/oder andere Manganverbindungen in einer Menge von 0,001 - 74,9 , vorzugsweise von 0,1 bis 50 Gew.%

enthält.

8. Verwendung des flüssigen Produktgemisches nach den Ansprüchen 1 - 7 als Motorkraftstoff oder Zusatz zu Motorkraftstoffen oder flüssigem Brennstoff.